# EUROPEAN PATENT SPECIFICATION

(11) **EP 3 307 865 B1**
(45) Date of publication and mention of the grant of the patent: **04.08.2021**
(21) Application number: 16731790.8
(22) Date of filing: 13.06.2016
(51) Int. Cl.: C12M 1/107, B01F 5/02, B01F 5/04, B01F 5/10, B01F 3/08, C12M 1/00, C12M 1/33

(54) **INJECTOR**
INJEKTOR
INJECTEUR

(30) Priority: 11.06.2015 DK 201570362
(43) Date of publication of application: 18.04.2018
(73) Proprietor: Landia A/S, 6940 Lem St (DK)
(72) Inventor: LARSEN, Steen Buhl, 6900 Skjern (DK)
(74) Representative: Patrade A/S
(86) International application number: PCT/DK2016/050179
(87) International publication number: WO 2016/198080

(56) References cited:
- WO-A1-2013/044926
- DE-A1- 3 309 834
- US-A- 3 271 304

## Description

### Field of the Invention

The present invention relates to a method as well as an apparatus for use in the inventive method, where the method fluidizes a slurry (both organic, semi-organic and non-organic slurries may be used) to such a degree that substantial advantages are obtained, both with respect to the potential bio gas yield from the treated organic slurry, but also energy savings throughout the processing and handling of the slurry.

These advantages are obtained with a relatively simple construction, which due to its construction provides reliable and low-cost treatment, yet resulting in substantial advantages.

### Background of the Invention

In the art it is known to shred organic materials comprising a substantially solid fraction and a liquid fraction in order to render available a larger surface for the microorganisms in the bioreactor to attack. Such examples are described in WO2013152771, WO2009150455 and WO 20113044926 A1 which discloses an apparatus to be installed on the wall of a biogas fermenter which comprises a shredder pump sucking an triturating the solids containing media which is inside the fermenter, the liquid mixture exiting the pump being further pumped through an injection nozzle and mixed with gas for obtaining a more homogeneous liquid containing fine particles which is again introduced in the tank.

However, the prior art has not recognized the advantages achievable by furthermore passing the shredded organic material through an injector device according to the present invention.

Also for other slurries, significant advantages may be obtained, which the present invention demonstrates.

### Object of the Invention

In order to improve the rentability of for example biogas production it is desirable to be able to process more organic material faster and to obtain a higher gas yield and also an improved quality of gas.

### Description of the Invention

The present invention addresses this need by providing a method for fluidization of a slurry as defined by claim 1.

Extensive testing has indicated that by firstly passing the organic slurry through the knife shredder and thereby making sure that particularly the solid fraction of the slurry has a maximum size, facilitates an increased reaction on both the liquid and the solid fraction as it passes the inverted funnel. As the material passes the funnel the pressure inside the material will diminish due to the increased area, i.e. the funnel shaped configuration of the funnel, such that the solid parts of the organic slurry will be more or less pulled apart.

Often the slurry processed in connection with biogas production is manure, which in addition to the normal fluid and substantially solid components may have an added content of straw or other cellulosic based material. Other slurries suitable to be treated with the present invention may be semi-organic slurries, for example sludge or tailings stemming from sewage treatment plants. Such semi-organic slurries will normally have a high protein content which may be a desirable resource for further processing, and which will be readily available after treatment in the inventive apparatus and method.

For biogas production it is also known to use corn, straw or other biomass containing materials as raw material. By shredding and passing the corn-slurry through the present invention and method, the resulting slurry is close to homogeneous in that the inventive method "pulls" the corn starch apart and exposes the lignin. This in turn facilitates easy and increased access for the bacteria in the biogas-reactor.

Furthermore, depending on the amount of tunnel flare (increase of diameter along the longitudinal/flow direction of the funnel, i.e. the difference between the area of the inlet and the outlet opening of the funnel, the under-pressure will also create vapour explosions inside the tiniest cells of the solid fraction thereby fractionizing the solid fractions to such a degree that a very fine and fragmented organic slurry exits the funnel.

Tests have indicated that the viscosity of the organic slurry is decreased by approx. 33% by passing it through the knife shredder and the funnel compared to a slurry which has not been subjected to the same treatment. In fact, a slurry having approx. 11% dry matter would have a viscosity of 9.61 cP in the untreated condition whereas the viscosity was measured at 6.6 cP after having been subjected to a method according to the present invention.

This is a significant number in that it provides for a number of advantages. First of all, the lower viscosity makes it easier to transport, i.e. to pump the slurry through a pipe system, such that both the volume of slurry which it is possible to transport in the pipe system may be increased and at the same time the energy needed to run the pumps may also be significantly lowered.

A further advantage which was discovered during the testing, was that without subjecting the slurry to the inventive method a biogas installation could handle between 400 and 450 kilos of added dry material (typically straw) per half hour having a pressure in the pumps of 6-7 bars whereas by introducing the same biomass material to the method according to the present invention, the dry matter could be increased to 600 kilos per half hour and at the same time lowering the pump pressure to 4-5 bars. This is a clear and significant difference afforded by the present invention. By increasing the amount of for example straw in the slurry, the lignin content in the slurry is increased. Lignin will stimulate and thereby increase the activity of the bacteria in the bioreactor vessel, resulting in an faster and increased gas production.

Furthermore, in addition to better viscosity and higher dry matter content the heat exchange coefficient of the slurry was also increased which led to an increased yield of particularly methane such that the yield without subjecting the slurry to the inventive method would be approximately 58%, but when the slurry was subjected to the inventive method the yield was raised to approx. 65% and more.

Consequently, it is clear that by subjecting an organic slurry to the inventive method substantial and significant advantages are achieved, which directly results in higher and better yields with less energy consumption.

In a further advantageous embodiment of the invention the inverted funnel projects into the pipe. In this manner the knife shredder and the funnel may be provided inside a pipe such that a stand-alone device may be created which may be inserted into the processing of organic slurries at any position in the process equipment.

In a still further advantageous embodiment a gas supply may be provided near the funnel's outlet, such that a gas is mixed into the slurry as it exits the funnel. By introducing a gas into the organic slurry as it leaves the funnel a larger volume will be introduced from the exit of the device. This is significant in that for example in embodiments where the method is applied to bioreactor tanks an increased stirring action will be created due to the larger volume of material being forced into the bioreactor. At the same time as the treated organic slurry leaves the funnel a further decrease in pressure will occur which will create a situation where the gas is thoroughly mixed inside the organic slurry thereby creating a substantially homogenous mass with a high reaction potential.

The invention is also directed at an apparatus as defined by claim 6.

In this connection it is particularly interesting to note the embodiments of the invention where the apparatus is constructed as a stand-alone device which may be inserted into a biomass reactor. The apparatus may also be advantageously used in other tanks such as for example pre-storage tanks, mixing tanks, (pre-)heating tanks etc. With respect to applying the apparatus in pre-storage tanks, the agitation created insuide the tank by the apparatus minimizes sedimentation inside the tank and consequently maintains more biological material in a slurry state which is easier to pump on to further processing. For mixing tanks it is self-evident that the improved consistency (less viscosity and more homogeneous) will make the mixing step better, faster and cheaper, resulting in higher yields etc. Typically the organic slurries will be heated prior to being introduced into the bio-reactor (or in the bio-reactor). When the inventive apparatus is introduced into heating tanks, the low viscosity and the relatively small particle sizes created by passage of the slurry through the apparatus, increases the heat-exchange ability of the slurry, resulting in faster and more homogeneous heating of the material.

This is particularly interesting in that in order to be able to treat the biomass materials through the inventive method according to the present invention it is not necessary to reconstruct or rebuild the entire process vessel, but simply to insert the stand-alone apparatus into the biomass will provide the advantages discussed above.

Also, it was found that by changing the characteristics of the funnel, i.e. the relationship between the inlet and outlet openings and the amount of taper corresponding to the length of the funnel, it was possible to influence the characteristics of the organic slurry which had been treated through the method and the apparatus.
Accordingly, the funnel is exchangeable, by means of a flange arranged around the inlet opening, said flange being adapted to be fitted against a supportive flange adjacent the outlet from the pump. The housing of the pump is therefore provided with a machined surface, where suitable means such as for example a groove and/or kerb is provided along with (threaded) bolt holes. In this manner a funnel having corresponding features (a groove corresponding to a kerb and/or a ridge/kerb corresponding with the groove etc). By arranging the funnel in this replaceable manner it is relatively easy to replace the funnel depending on use, simply by unscrewing bolts and removing the funnel in place and replacing a new/another suitable funnel.

The funnel suitable for the specific slurry to be treated may be selected and mounted, where the funnels to be selected amongst will have variations in one or more or combinations of the following:
When the openings are circular:
a. Relative diameter between inlet orifice d₁ and outlet opening d₂, where said relative diameters have a ratio (d₁/d₂) between 0,95 to 0,15
b. distance 1 between inlet and outlet is selected between 5 mm and 250 mm when the openings are non-circular:
c. the relative cross-sectional areas between inlet orifice and outlet opening has a ratio between 0,95 to 0,15
d. distance 1 between inlet orifice and outlet opening is selected between 5 mm and 250 mm.
Usually the funnel will have circular cros-sections but other cross-sectional shapes are also contemplated. For example oval, rectangular, square and other suitable shapes.

Further parameters which may be selected depending on the slurry to be treated may be combinations or individual parameters selected from the funnels inlet orifice has a first area and where the outlet has an outlet area, and where the inlet opening is spaced a distance 1 from the outlet opening, and where:
- When the inlet orifice and the outlet are circular:
   - d₁ is between 40 mm and 250 mm;
   - d₂ is selected between 45 mm and 300 mm;
   - 1 is selected between 5 mm and 250 mm.
   when the inlet orifice and the outlet are non-circular:
   - the inlet orifice is between 1200 mm² and 50000 mm²
   - the outlet area is between 1400 mm² and 70000 mm²
   - 1 is selected between 5 mm and 250 mm.

The parameters selected depended on the constituents of the organic fluid such that for example for organic material having a relatively high solid fraction the taper was selected to be relatively steep, i.e. the difference between inlet opening and outlet opening of the funnel would be substantially larger as for slurry containing a smaller amount of solid material. Also the length of the funnel is important to the resulting treatment and as such for particularly organic material having a high solid fraction content the longer funnel would be advantageous in that it would provide a longer and more consistent period of treatment at a lower pressure thereby providing more time for the process/method to tear the solid fraction of the biological material apart.

In other instances it might be advantageous to mount the device directly in the wall of the bioreactor such that it is possible to service the device from the outside and in instances where gas is introduced after the funnel the gas may be introduced from an outside source or from a different bioreactor in order to create a thorough mix.

Further advantageous embodiments are disclosed in the further dependent claims.

The present inventive method and apparatus particularly finds use in connection with biogas manufacturing plants, particularly in addition to reducing the viscosity which provides increased pumpability, better heat-exchange, increased bio-activity and thereby also increased and faster gas production, it also provides mixing of the contents of the bio-reactor vessels contents. The mixing ability is also important for sewage treatment plants where the mixing aspect of the present invention increases the homogenisation of the sludge/sewage.

The manure (biomass) used in many instances as raw material for the biogas production, may also before it is introduced into the bio-reactors vessels be treated according to the invention. Thereby is achieved that the material handling is more efficient particularly due to the reduced viscosity, which eases the handling and pumping of the manure, and at the same time, readies the material for the bio-treatment in the bio-gas treatment reactor, again increasing the yield.

In some farm/industrial and bio-reactor lay-outs it is necessary to pump slurries of the types mentioned above (in particular biomass and especially manure) long distances. By treating the raw material, i.e. the slurry according to the method of the present invention, it becomes possible with less energy use to transport the manure over longer distances.

### Description of the Drawing

The invention will now be described with reference to the accompanying drawings wherein
- Figure 1: illustrates an embodiment of the invention
- Figure 2: illustrates a slurry containing vessel 50 which is equipped with an injector according to the present invention
- Figure 3: illustrates a schematic installation of an apparatus according to the present invention
- Figure 4: illustrates a cross-section through the part of the injector where the funnel 70 is arranged.

### Detailed Description of the Invention

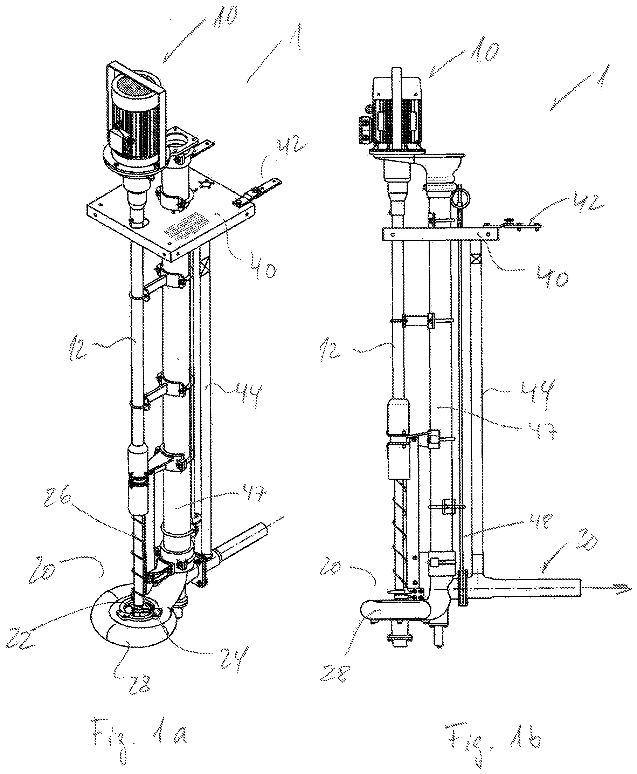

In figure 1a and 1b is illustrated an embodiment of the invention where the apparatus which will hereafter be referred to as the injector is constructed such that it may be submerged into a liquefied biomass. In this embodiment the injector comprises a motor 10 which by means of a drive shaft 12 is connected to an injector 20 according to the present invention.

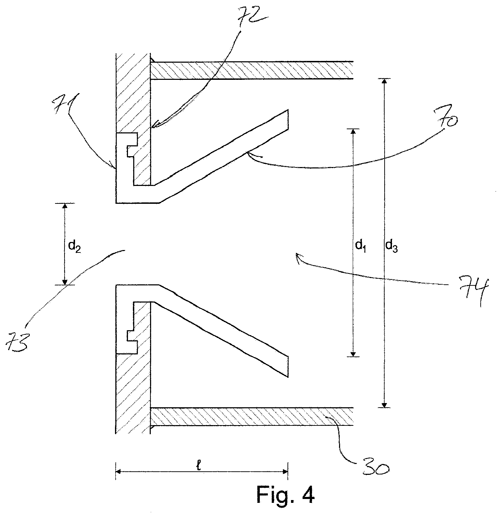

The injector 20 comprises an inlet 22. In the inlet 22 is arranged a set of shredder knifes 24 and furthermore in this embodiment a helical structure 26 is provided on a lower section of the drive axle 12 such that more solid material will be urged downwards and into the inlet 22 of the injector 20. The shredder knives 24 will divide the solid parts of the slurry which enters into the injector prior to a pump 28 increasing the pressure in the liquid which is entered through the inlet 22. A funnel (not illustrated in figures 1a and 1b) is inserted after the pump 28 and before the injection pipe 30. The funnel is explained with reference to figure 4, see below.

Once the slurry has passed the funnel it is typically introduced into the injector pipe 30 which again introduces the liquefied slurry into the material inside the tank in which the device 1 is mounted.

The embodiment illustrated in figures 1a and 1b is provided with a mounting platform 40 which has suitable brackets 42 such that the mounting platform 40 can easily be mounted to a top section of a slurry containing vessel.

Also provided is a gas inlet pipe 44 such that for example biogas may be sucked into the slurry flow as it leaves the outlet of the funnel. Other gas types such as CO₂, H₂, air and others may also be introduced depending on the particular slurry being treated and the object of the treatment. Due to the difference in pressure, i.e. after the slurry has passed the funnel and is directed into the injector pipe 30, a pressure drop will occur which will allow for a gas injected through the pipe 44 to mix completely with the slurry such that the injected mixture of treated slurry and gas will have a relatively large volume and a high velocity such that the impact on the remaining slurry in the tank may be quite substantial.

In this particular embodiment it is possible to select between circulation in the tank, and pumping slurry out of the tank. By manipulating the valve 48 it is possible to select that the chopped/shredded liquid is either directed to the injector 30, or led into the pipe 47, which will pump liquid out of the tank.

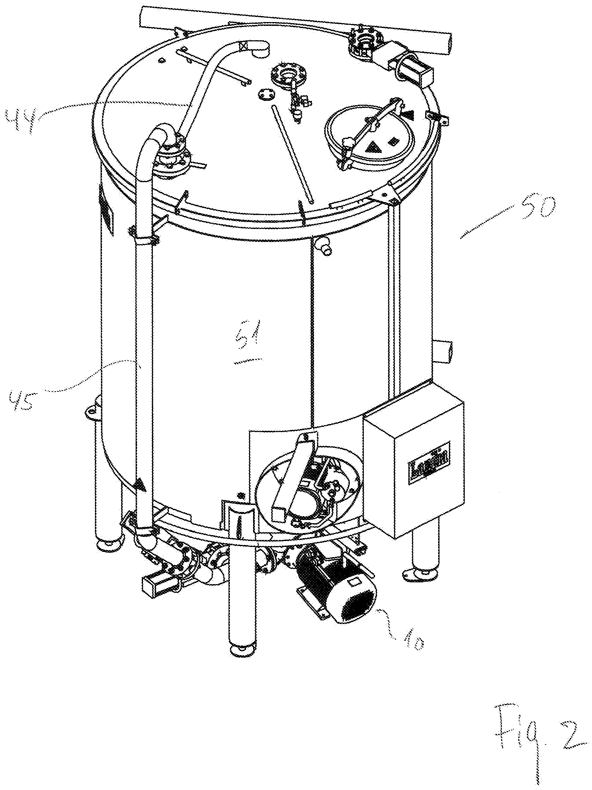

In figure 2 is illustrated a slurry containing vessel 50 which is equipped with an injector according to the present invention. The pump motor 10 is arranged below the tank where also the shredder pump is installed. A gas supply 44 is connected in the same manner as described above with reference to figures 1a and 1b. In this embodiment the injector pipe (not visible) is arranged in the wall 51 of the vessel 50 and may be oriented relative to the wall 51 such that the resulting injection of treated slurry is injected for example along the wall 51 of the vessel 50 in order to create a rotation and thereby a mixture inside the vessel 50. Therefore, in addition to treating the slurry as described above also a further rotation and thereby mix of the slurry inside the vessel is achieved during the same process.

As was the case in the embodiment described above, the tank/vessel 50 is also provided with a possibility to pump liquid directly into the tank (or another vessel, but illustrated as pumping into the same vessel) by directing the liquid/slurry into pipe 45.

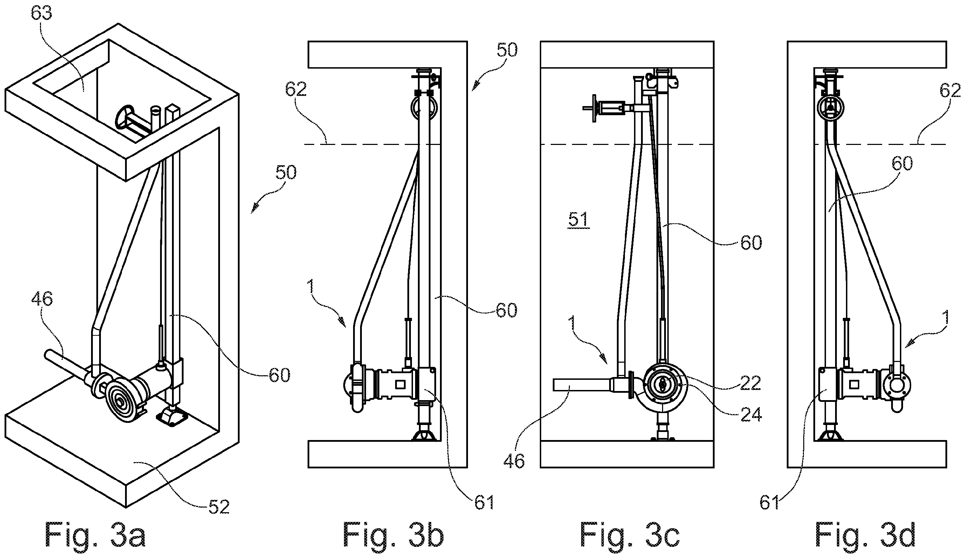

Turning to figure 3 a schematic installation of an apparatus according to the present invention inside a slurry containing vessel 50 is illustrated. The injector 1 is fastened to a column 60 by a bracket construction 61. The column 60 is fastened to the wall 51 and bottom 52 of the vessel 50 such that the injector will remain in place when activated. The inlet 22 is in this embodiment oriented such that the shredder knives 24 are rotated about a horizontal axis, but other orientations are also contemplated as is for example illustrated in figure 1a where the rotation axis is vertical. The injector pipe 46 is arranged to eject treated slurry along the wall 51 of the vessel 50 in order to create rotation as was the case with the arrangement as depicted in figure 2.

The bracket 61 which mounts the injector 1 to the column 60 is in this embodiment designed to allow the injector 1 to slide vertically along the column 60 such that for example for service purposes it is possible to elevate the pump outside the liquid, i.e. the surface of the liquid is illustrated with a dashed line 62. By further arranging the column adjacent an opening 63 in an upper part of the vessel 50 it is possible to either install the injector with the column 60 in already existing tanks or to withdraw the injector for service through the opening 63.

Turning to figure 4 which illustrates a cross-section through the part of the injector where the funnel 70 is arranged. The funnel 70 is in this embodiment a machine-worked, replaceable funnel which has engagement flanges 71 such that it may be arranged in an opening provided in the injector by being fitted with receiving flanges 72. In this manner a very easy and safe as well as stable mounting of the funnel in the device is achieved. The funnel has a length 1 limited in both ends by an inlet opening 73 and an outlet opening 74.

The ratio between d₁ and d₂ as well as the relative length between d₁ and d₂ is important in order to achieve the desired fluidization of the slurry passing through the funnel 70.

Furthermore, in this embodiment the funnel 70 is arranged such that the outlet opening 74 is inside the injector pipe 30. As slurry is passed under pressure through the funnel 70 towards the right in figure 4 an under-pressure will occur adjacent and outside the outlet opening 74. This sudden drop in pressure will cause the material in the slurry to expand rapidly thereby disintegrating the more solid particles of the slurry thus creating a more liquefied slurry for further processing. It is also in this position that the injector pipe may be provided with a gas inlet as illustrated in figures 1a and 1b where gas inlet 44 is indicated as being attached through the injector pipe 30 in the vicinity of the funnel's outlet 74.

In embodiments of the invention where it is possible to retrieve the injector 1 from the slurry containing vessel, the funnel 70 may be replaced for example due to wear or where the ration between d₁ and d₂ and possibly the length 1 may vary depending on the constituents and viscosity of the slurry which is to be treated with the inventive device according to the present invention. Replacement of the funnel may very easily take place simply by dismantling part of the injector and replacing one funnel element 70 by another more suitable funnel element.

Tests have indicated that the ratio between d₁ and d₂ is important in that the bigger the difference, the more compressed the slurry will become passing through the funnel and thereby the greater the impact of the under-pressure as it is released into the relatively larger space indicated by d₃ designating the interior diameter of the injector pipe 30.

The increased under-pressure arising after the outlet 74 of the funnel 70 will cause internal expansion in the slurry material and also increased vapour explosions thereby disintegrating the more solid particles of the slurry material.

## Claims

1. Method for fluidization of a slurry comprising a fluid fraction and a solid fraction, where the slurry is first pumped through a knife shredder pump and secondly passed through an inverted funnel (70) having an inlet orifice (73) having a first cross-sectional area and an outlet (74) having a cross-sectional outlet area, where the outlet (74) is larger than the inlet orifice (73), wherein the funnel (70) is exchangeable, by means of a flange (71) arranged around the inlet opening (73), said flange (71) being adapted to be fitted against a supportive flange (72) adjacent the outlet from the pump (28), such that a funnel (70) suitable for the specific slurry to be treated may be selected and mounted, where the slurry is injected into a pipe (30) having a cross-sectional area larger than the outlet area, such that the slurry is subjected to a substantial under-pressure upon leaving the outlet (73).

2. Method according to claim 1, wherein the inverted funnel (70) projects into the pipe (30).

3. Method according to claim 1 or claim 2, wherein further gas is mixed into the slurry after the slurry is introduced into the funnel (70), by means of a gas supply (44) provided near the funnel's outlet (74).

4. Method according to any preceding claim wherein the knife shredder pump is dimensioned such that it will be able to suck the slurry into the pump and after shredding the slurry, convey the slurry through the funnel (70).

5. Method according to any preceding claim wherein the slurry is manure, biomass, sludge, sewage, clay.

6. Apparatus suitable to carry out the method according to any of claims 1 to 5, where said apparatus comprises:
a. an inlet (22) to
b. a pump (20);
c. one or more shredding knives (24) arranged in or before the pump (20), said pump having an outlet (28);
d. a funnel (70) having an inlet orifice(73) facing the outlet (28) of the pump (20) and an outlet opening (74) facing away from the pump (20), where the inlet opening (73) is smaller than the outlet opening (74);
e. an injector pipe (30) surrounding at least the outlet (74) of the funnel (70) and wherein the funnel (70) is exchangeable, by means of a flange (71) arranged around the inlet opening (73), said flange (71) being adapted to be fitted against a supportive flange (72) adjacent the outlet (28) from the pump (20), such that a funnel (70) suitable for the specific slurry to be treated may be selected and mounted.

7. Apparatus according to claim 6, wherein drive means (10) for the pump (20) and the one or more shredding knives (24) is arranged at a distance from the pump (20) and knives (24).

8. Apparatus according to claim 6 or 7, wherein at least the inlet orifice (73), pump (20), shredding knives (24), funnel (70) and injector pipe (30,46) is suitable to be fully immersed into the organic slurry.

9. Apparatus according to claim 6 or 7, wherein only the injector pipe (30,46) is suitable to be fully immersed or in contact with the organic slurry, and where means are provided for mounting the apparatus on a wall of a vessel containing an organic slurry, such that the injector pipe (30,46) projects into the organic slurry.

10. Apparatus according to any of claims 6 to 9, wherein the funnels (70) to be selected amongst will have variations in one or more or combinations of the following:
When the openings are circular:
a. Relative diameter between inlet orifice d₁ and outlet opening d₂, where said relative diameters have a ratio d₁/d₂ between 0,95 to 0,15
b. distance 1 between inlet and outlet is selected between 5 mm and 250 mm when the openings are non-circular:
c. the relative cross-sectional areas between inlet orifice and outlet opening has a ratio between 0,95 to 0,15
d. distance 1 between inlet orifice and outlet opening is selected between 5 mm and 250 mm.

11. Apparatus according to claim 8 or 10, wherein the funnels inlet orifice (73) has a first area and where the outlet has an outlet area, and where the inlet opening (73) is spaced a distance 1 from the outlet opening (74), and where:
- When the inlet orifice (73) and the outlet (74) are circular:
- d₁ is between 40 mm and 250 mm;
- d₂ is selected between 45 mm and 300 mm;
- 1 is selected between 5 mm and 250 mm.
when the inlet orifice (73) and the outlet (74) are non-circular:
- the inlet orifice is between 1200 mm² and 50000 mm²
- the outlet area is between 1400 mm² and 70000 mm²
- l is selected between 5 mm and 250 mm.

## Patentansprüche

1. Verfahren zur Fluidisierung eines Schlamms, der eine Fluidfraktion und eine Feststoff-fraktion umfasst, wobei der Schlamm zunächst durch eine Schredderpumpe mit Messern gepumpt wird und danach durch einen umgedrehten Trichter (70) mit einer Einlassöffnung (73), die einen erste Querschnittsbereich aufweist, und einem Auslass (74), der einen Querschnittsauslassbereich aufweist, geleitet wird, wobei der Auslass (74) größer als die Einlassöffnung (73) ist, wobei der Trichter (70) mittels eines Flansches (71), der um die Einlassöffnung (73) herum angeordnet ist, austauschbar ist, wobei der Flansch (71) angepasst ist, um an einem unterstützenden Flansch (72) angrenzend an den Auslass von der Pumpe (28) befestigt zu werden, sodass ein Trichter (70), der für den zu behandelnden spezifischen Schlamm geeignet ist, ausgewählt und montiert werden kann, wobei der Schlamm in ein Rohr (30) mit einem Querschnittsbereich, der größer als der Auslassbereich ist, injiziert wird, sodass der Schlamm beim Herausströmen aus dem Auslass (73) einem wesentlichen Unterdruck ausgesetzt ist.

2. Verfahren nach Anspruch 1, wobei der umgedrehte Trichter (70) in das Rohr (30) hineinragt.

3. Verfahren nach Anspruch 1 oder 2, wobei ferner Gas in den Schlamm gemischt wird, nachdem der Schlamm mittels einer Gaszufuhr (44), die in der Nähe des Auslasses (74) des Trichters bereitgestellt ist, in den Trichter (70) eingeleitet wird.

4. Verfahren nach einem der vorhergehenden Ansprüche, wobei die Schredderpumpe mit Messern so dimensioniert ist, dass sie in der Lage sein wird, den Schlamm in die Pumpe zu saugen und den Schlamm nach dem Schreddern des Schlamms durch den Trichter (70) zu befördern.

5. Verfahren nach einem der vorhergehenden Ansprüche, wobei der Schlamm Dung, Biomasse, Klärschlamm, Abwasser, Lehm ist.

6. Vorrichtung, die zum Ausführen des Verfahrens nach einem der Ansprüche 1 bis 5 geeignet ist, wobei die Vorrichtung Folgendes umfasst:
a. einen Einlass (22) zu
b. einer Pumpe (20);
c. ein oder mehrere Schreddermesser (24), die in oder vor der Pumpe (20) angeordnet sind, wobei die Pumpe einen Auslass (28) aufweist;
d. einen Trichter (70) mit einer Einlassöffnung (73), die dem Auslass (28) der Pumpe (20) zugewandt ist, und einer Auslassöffnung (74), die der Pumpe (20) abgewandt ist, wobei die Einlassöffnung (73) kleiner als die Auslassöffnung (74) ist;
e. ein Injektorrohr (30), das mindestens den Auslass (74) des Trichters (70) umgibt, und wobei der Trichter (70) mittels eines Flansches (71), der um die Einlassöffnung (73) herum angeordnet ist, austauschbar ist, wobei der Flansch (71) angepasst ist, um an einem unterstützenden Flansch (72) angrenzend an den Auslass (28) von der Pumpe (20) befestigt zu werden, sodass ein Trichter (70), der für den zu behandelnden spezifischen Schlamm geeignet ist, ausgewählt und montiert werden kann.

7. Vorrichtung nach Anspruch 6, wobei ein Antriebsmittel (10) für die Pumpe (20) und das eine oder die mehreren Schreddermesser (24) in einem Abstand von der Pumpe (20) und den Messern (24) angeordnet sind.

8. Vorrichtung nach Anspruch 6 oder 7, wobei mindestens die Einlassöffnung (73), Pumpe (20), Schreddermesser (24), der Trichter (70) und das Injektorrohr (30,46) geeignet sind, um vollständig in den organischen Schlamm eingetaucht zu werden.

9. Vorrichtung nach Anspruch 6 oder 7, wobei nur das Injektorrohr (30,46) geeignet ist, um vollständig in den organischen Schlamm eingetaucht zu werden oder in Kontakt mit diesem zu sein, und wobei Mittel zur Montage der Vorrichtung an einer Wand eines Behälters, der einen organischen Schlamm enthält, bereitgestellt sind, sodass das Injektorrohr (30,46) in den organischen Schlamm hineinragt.

10. Vorrichtung nach einem der Ansprüche 6 bis 9, wobei die Trichter (70), aus denen auszuwählen ist, Variationen bei einer oder mehreren oder Kombinationen der Folgenden aufweisen:
Wenn die Öffnungen kreisförmig sind:
a. Relativer Durchmesser zwischen Einlassöffnung d₁ und Auslassöffnung d₂, wobei die relativen Durchmesser ein Verhältnis von d₁:d₂ von zwischen 0,95 bis 0,15 aufweisen
b. wird Abstand l zwischen Einlass und Auslass zwischen 5 mm und 250 mm ausgewählt, wenn die Öffnungen nicht kreisförmig sind:
c. weisen die relativen Querschnittsbereiche zwischen Einlassöffnung und Auslassöffnung ein Verhältnis von zwischen 0,95 bis 0,15 auf
d. wird Abstand l zwischen Einlassöffnung und Auslassöffnung zwischen 5 mm und 250 mm ausgewählt.

11. Vorrichtung nach Anspruch 8 oder 10, wobei die Einlassöffnung (73) der Trichter einen ersten Bereich aufweist und der Auslass einen Auslassbereich aufweist, und wobei die Einlassöffnung (73) einen Abstand l von der Auslassöffnung (74) beabstandet ist, und wobei:
- Wenn die Einlassöffnung (73) und der Auslass (74) kreisförmig sind:
- d₁ zwischen 40 mm und 250 mm beträgt;
- d₂ von zwischen 45 mm und 300 mm ausgewählt wird;
- l von zwischen 5 mm und 250 mm ausgewählt wird.
wenn die Einlassöffnung (73) und der Auslass (74) nicht kreisförmig sind:
- die Einlassöffnung zwischen 1200 mm² und 50000 mm² beträgt
- der Auslassbereich zwischen 1400 mm² und 70000 mm² beträgt
- l von zwischen 5 mm und 250 mm ausgewählt wird.

## Revendications

1. Procédé de fluidisation d'une suspension comprenant une fraction liquide et une fraction solide, où la suspension est tout d'abord pompée à travers une pompe broyeuse à couteaux puis passée à travers un entonnoir inversé (70) ayant un orifice d'entrée (73) présentant une première surface en coupe transversale et une sortie (74) présentant une surface de sortie en coupe transversale, où la sortie (74) est plus large que l'orifice d'entrée (73), dans lequel l'entonnoir (70) est amovible, au moyen d'une bride (71) agencée autour de l'ouverture d'entrée (73), ladite bride (71) étant conçue pour être montée contre une bride de support (72) adjacente à la sortie de la pompe (28), de sorte qu'un entonnoir (70), adapté à la suspension spécifique à traiter, peut être choisi et monté, où la suspension est injectée dans un tuyau (30) présentant une surface en coupe transversale supérieure à la surface de sortie, de sorte que la suspension est soumise à une importante dépression lors de l'expulsion par la sortie (73).

2. Procédé selon la revendication 1, dans lequel l'entonnoir inversé (70) fait saillie dans le tuyau (30).

3. Procédé selon la revendication 1 ou la revendication 2, dans lequel du gaz supplémentaire est mélangé dans la suspension après que la suspension a été introduite dans l'entonnoir (70), au moyen d'une alimentation en gaz (44) prévue à proximité de la sortie de l'entonnoir (74).

4. Procédé selon une quelconque revendication précédente, dans lequel la pompe broyeuse à couteaux est dimensionnée de sorte qu'elle pourra aspirer la suspension dans la pompe puis, après le broyage de la suspension, acheminer la suspension à travers l'entonnoir (70).

5. Procédé selon une quelconque revendication précédente, dans lequel la suspension est du fumier, de la biomasse, de la boue, des eaux usées, de l'argile.

6. Appareil adapté pour mettre en œuvre le procédé selon l'une quelconque des revendications 1 à 5, ledit appareil comprenant :
a. une entrée (22) vers
b. une pompe (20) ;
c. un ou plusieurs couteaux de broyage (24) agencés à l'intérieur de la pompe (20) ou avant celle-ci, ladite pompe ayant une sortie (28) ;
d. un entonnoir (70) ayant un orifice d'entrée (73) faisant face à la sortie (28) de la pompe (20) et une ouverture de sortie (74) opposée à la pompe (20), où l'ouverture d'entrée (73) est plus étroite que l'ouverture de sortie (74) ;
e. un tuyau d'injection (30) entourant au moins la sortie (74) de l'entonnoir (70) et
dans lequel l'entonnoir (70) est amovible, au moyen d'une bride (71) agencée autour de l'ouverture d'entrée (73), ladite bride (71) étant conçue pour être montée contre une bride de support (72) adjacente à la sortie (28) de la pompe (20), de sorte qu'un entonnoir (70), adapté à la suspension spécifique à traiter, peut être choisi et monté.

7. Appareil selon la revendication 6, dans lequel un moyen d'entraînement (10) pour la pompe (20) et les un ou plusieurs couteaux de broyage (24) est agencé à distance de la pompe (20) et des couteaux (24).

8. Appareil selon la revendication 6 ou 7, dans lequel au moins l'orifice d'entrée (73), la pompe (20), les couteaux de broyage (24), l'entonnoir (70) et le tuyau d'injection (30, 46) sont adaptés pour être complètement immergés dans la suspension organique.

9. Appareil selon la revendication 6 ou 7, dans lequel seul le tuyau d'injection (30, 46) est adapté pour être complètement immergé ou en contact avec la suspension organique, et où des moyens sont prévus pour monter l'appareil sur une paroi d'un récipient contenant une suspension organique, de sorte que le tuyau d'injection (30, 46) fait saillie dans la suspension organique.

10. Appareil selon l'une quelconque des revendications 6 à 9, dans lequel les entonnoirs (70) parmi lesquels choisir présenteront des variations dans un ou plusieurs ou dans des combinaisons des éléments suivants : lorsque les ouvertures sont circulaires :
a. un diamètre relatif entre l'orifice d'entrée d₁ et l'ouverture de sortie d₂, lesdits diamètres relatifs ayant un rapport d₁/d₂ compris entre 0,95 et 0,15
b. la distance 1 entre l'entrée et la sortie est sélectionnée entre 5 mm et 250 mm lorsque les ouvertures sont non circulaires :
c. les surfaces en coupe transversale relatives entre l'orifice d'entrée et l'ouverture de sortie ont un rapport compris entre 0,95 et 0,15
d. la distance 1 entre l'orifice d'entrée et l'ouverture de sortie est sélectionnée entre 5 mm et 250 mm.

11. Appareil selon la revendication 8 ou 10, dans lequel l'orifice d'entrée (73) de l'entonnoir présente une première surface et où la sortie présente une surface de sortie, et où l'ouverture d'entrée (73) est espacée de l'ouverture de sortie (74) selon une distance 1, et où :
- lorsque l'orifice d'entrée (73) et la sortie (74) sont circulaires :
- d₁ est compris entre 40 mm et 250 mm ;
- d₂ est sélectionné entre 45 mm et 300 mm ;
- 1 est sélectionné entre 5 mm et 250 mm.
lorsque l'orifice d'entrée (73) et la sortie (74) sont non circulaires :
- l'orifice d'entrée est compris entre 1 200 mm² et 50 000 mm²
- la surface de sortie est comprise entre 1 400 mm² et 70 000 mm²
- 1 est sélectionné entre 5 mm et 250 mm.
